# EUROPEAN PATENT SPECIFICATION

(11) **EP 3 992 628 B1**
(45) Date of publication and mention of the grant of the patent: **07.01.2026**
(21) Application number: 21205434.0
(22) Date of filing: 29.10.2021
(51) Int. Cl.: G01N 33/03, G01N 31/22

(54) **PROCESS AND APPARATUS FOR EVALUATING THE TEMPORAL VALIDITY OF THE HEALTH RECOMMENDATIONS OF OLIVE OIL**
VERFAHREN UND VORRICHTUNG ZUM AUSWERTEN DER ZEITLICHEN GÜLTIGKEIT VON GESUNDHEITSEMPFEHLUNGEN VON OLIVENÖL
PROCÉDÉ ET DISPOSITIF POUR ÉVALUER LA VALIDITÉ TEMPORELLE DES RECOMMANDATIONS SANITAIRES DE L'HUILE D'OLIVE

(30) Priority: 02.11.2020 IT 202000026050
(43) Date of publication of application: 04.05.2022
(73) Proprietor: Università degli Studi di Bari "Aldo Moro", 70121 Bari (BA) (IT)
(72) Inventor: CLODOVEO, Maria Lisa, 70126 Bari (BA) (IT); CORBO, Filomena Faustina Rina, 70020 Bitritto (BA) (IT)
(74) Representative: Trupiano, Federica

(56) References cited:
- CALABRIA D ET AL: "Paper-based smartphone chemosensor for reflectometric on-site total polyphenols quantification in olive oil", SENSORS AND ACTUATORS B: CHEMICAL, ELSEVIER BV, NL, vol. 305, 2 December 2019 (2019-12-02), XP085968858, ISSN: 0925-4005, [retrieved on 20191202], DOI: 10.1016/J.SNB.2019.127522
- HIDAYAT MOCHAMMAD AMRUN ET AL: "Development of Paper Based Sensor for the Determination of Total Phenolic Content in Green Tea Beverages", AGRICULTURE AND AGRICULTURAL SCIENCE PROCEDIA, ELSEVIER, AMSTERDAM, NL, vol. 9, 23 February 2016 (2016-02-23), pages 424 - 430, XP029440479, ISSN: 2210-7843, DOI: 10.1016/J.AASPRO.2016.02.159
- MAR�A JOS� MOYANO ET AL: "The Color of Olive Oils: The Pigments and Their Likely Health Benefits and Visual and Instrumental Methods of Analysis The Importance of Olive Oil Color: Not Only a Matter of Acceptability", 1 September 2010 (2010-09-01), pages 1 - 15, XP055815620, Retrieved from the Internet <URL:https://onlinelibrary.wiley.com/doi/full/10.1111/j.1541-4337.2010.00109.x> [retrieved on 20210618]
- BASTIDA SARA ET AL: "Colorimetric test application to the efficiency and shelf-life assessments of olive oil, sunflower oil and their blend used for frying frozen and fresh foods", GRASAS Y ACEITES, vol. 54, no. 1, 30 March 2003 (2003-03-30), SPAIN, XP055815619, ISSN: 0017-3495, DOI: 10.3989/gya.2003.v54.i1.274
- GARCIA BARBARA ET AL: "A simple method for the determination of bioactive antioxidants in virgin olive oils : A simple method for the determination", JOURNAL OF THE SCIENCE OF FOOD AND AGRICULTURE, vol. 93, no. 7, 29 November 2012 (2012-11-29), GB, pages 1727 - 1732, XP055815618, ISSN: 0022-5142, DOI: 10.1002/jsfa.5958
- ZANONI BRUNO ET AL: "A preliminary approach to predictive modelling of extra virgin olive oil stability", JOURNAL OF THE SCIENCE OF FOOD AND AGRICULTURE, vol. 85, no. 9, 1 January 2005 (2005-01-01), GB, pages 1492 - 1498, XP055815611, ISSN: 0022-5142, DOI: 10.1002/jsfa.2135

## Description

### STATE OF THE ART

According to the WHO, about 1/3 of the cardiovascular diseases and cancers could be prevented by a healthy and balanced diet. The full awareness of the link between diet and health stimulates the consumer's need to know the composition of what he/she takes into his/her body. The consumer himself/herself today seeks guarantees regarding the absence of harmful molecules and in relation to the presence of components with beneficial and functional action in the food to be consumed. In order to avoid cases of misleading advertising, based on healthy information without scientific basis but able to influence consumers' food choices and habits, the European Parliament approved the 1924/2006 Regulation, which regulates the nutritional and healthy recommendations/claims provided on the food products.

Said nutritional and healthy recommendations/claims provided on the food products are known as "claims". In the present invention, the expressions "healthy recommendation/claim", "healthy recommendations/claims" or "recommendations/claims for the health" are used interchangeably to refer to the approved recommendation/claims of the Regulation 1924/2006.

A particularly important healthy recommendation for the olive oil relates to the polyphenol content. The polyphenols are chemical compounds known for their antioxidant and anti-inflammatory properties and present in most plant-based foods. In order for 5 mg of bio-phenols to be taken per day, which is the minimum quantity required to correctly carry out the antioxidant action, and considering an average daily consumption of 20 g of olive oil, it is necessary that the concentration of bio-phenols in the product is greater than or equal to 250 mg/kg (total polyphenols at least greater than or equal to 300 mg/kg) until the minimum shelf-life. In order to ensure the validity of the healthy recommendation concerning polyphenols in the olive oil for at least 18 months from the bottling date, the initial bio-phenol content should be at least twice the minimum quantity required, as polyphenols in the olive oil undergo oxidation reactions and therefore degradation.

The polyphenols are chemical substances characterized by one or more hydroxyl groups on the benzene ring that exert a protective action on the oxidation of the oil by the oxygen. As numerous studies demonstrate, they also have a powerful antioxidant action on our body. The bio-phenols or bio-active phenols are the phenols within the olive oil that have antioxidant activity.

One of the main obstacles encountered in the use of the healthy recommendations in relation to the polyphenols is the lack of a reliable predictive model for defining the length of time the healthy recommendation remains valid, depending on the initial concentration of bio-phenols, the selected packaging and the storage conditions. The techniques used to date to evaluate the polyphenol content in the oil, in order to apply on the label the healthy recommendation related to the polyphenols and the expiry date of the product, are based on sophisticated methods of analysis that require expensive equipment, high professional specialization and specific skills.

Calabria et Al. "Paper based smartphone chemosensor for reflectometric on-site total polyphenols quantification in olive oil", Sensor and actuators B: Chemical, Elsevier BV, NL, vol. 305, 02.12.2019, XP085968858, describes a paper-based chemosensor, based on the colorimetric assay known as the Folin-Ciocalteu assay (FC), for the rapid quantitative determination of polyphenols in extra virgin olive oils (EVOO) without preliminary samples extraction. The protocol of the FC assay described has been optimized for the detection of images via smartphone both by maximizing the blue-grey color obtained in the presence of polyphenols, and by minimizing the yellow background color determined by the unreacted FC reagent. This method is based on the objective of carrying out a quantitative determination of the total content of polyphenols in EVOO, without the need to carry out preventive extractions on the EVOO sample to be analyzed.

High performance liquid chromatography (HPLC) coupled with a mass spectrometry or ultraviolet detector is one of the techniques used to evaluate the polyphenol content in the olive oil. However, this technique makes use of very expensive analytical instruments and requires qualified technicians. Furthermore, said high-performance liquid chromatography (HPLC) coupled with a mass spectrometry or ultraviolet detector returns a parameter, i.e. the polyphenol content expressed in mg/kg of oil, which does not allow the operators in the olive oil field to easily discriminate oils with a polyphenol concentration compatible with the healthy recommendations. In other words, said technique does not return a value of time validity of the healthy recommendation related to the polyphenols in the olive oil, and therefore does not allow to establish the shelf-life of the product, i.e. its duration of preservation such that the statements on the label, including the healthy recommendations, remain unchanged. The complexity of the composition of the olive oils and their considerable variability makes it difficult to predict how the quality of the product will evolve and what its actual shelf-life will be, also in relation to the packaging methods and environmental storage conditions. Therefore, there's a need for a process that allows to evaluate and/or predict the temporal validity of the healthy recommendations related to the polyphenols of the olive oil, preferably extra-virgin olive oil.

### OBJECTS OF THE INVENTION

An object of the present invention is to provide a process according to claim 1 for evaluating the temporal validity of the healthy recommendations related to the polyphenols of the olive oil and in particular extra-virgin olive and to provide the capability of determining the duration of preservation of an oil sample, i.e. its shelf-life, in relation also to the packaging methods and environmental storage conditions.

Still an object of the present invention is to provide an apparatus according to claim 9 allowing to evaluate the temporal validity of the healthy recommendations related to the polyphenols of the olive oil and in particular extra-virgin olive oil.

Said apparatus does not require complex equipment and high professional technical skills, while allowing to evaluate the temporal validity of the healthy recommendations related to the polyphenols of the olive oil and in particular extra-virgin olive oil.

### DESCRIPTION OF THE INVENTION

The above purposes, as well as other purposes, are achieved by means of the object of the present invention, namely a process for evaluating the temporal validity of the healthy recommendations related to the polyphenols present in a sample of olive oil, comprising the steps of:
a) obtaining color data of the polyphenol level in said sample of virgin vegetable oil by colorimetric chemical assay, said color data being quantitative data of the level of said polyphenols in said oil sample;
b) reading the intensity of said color data of step a) by an electronic device provided with a camera; and
c) processing the data of step b) by a predictive model adapted to determine the minimum term of validity of the healthy recommendation of said oil sample.

The process of the invention relates to evaluating the temporal validity of the healthy recommendation related to the polyphenols present a sample of virgin vegetable oil, more preferably an extra-virgin olive oil.

In the present invention, the expressions "healthy recommendation/claim" or "healthy recommendations/claims" both refer to the term "claim" according to the Regulation 1924/2006.

The healthy and nutritional recommendations, which can be made explicit on food labels and/or through advertising, are the set of nutritional/health guarantee recommendations, i.e. recommendations related to possible positive effects on health. Said recommendations must therefore be easily understandable by the consumer and must demonstrate, on the basis of scientific evidence, that the nutrient has nutritional or beneficial effect that it is present in significant amounts to obtain the stated effect in the food product, that can have the stated effect when a reasonable amount of the food product is consumed and that it is present in a form usable by the body.

In particular, the European Parliament with the Regulation 1924/2006 recognizes two types of recommendations/claims:
- nutritional recommendations, i.e. recommendations which relate to particular beneficial nutritional properties due to the energy (caloric value) which the mentioned food product provides/does not provide, and/or to the nutrients or other substances it contains/does not contain;
- healthy recommendations, i.e. recommendations which relate to the existence of a direct relationship between a food or a food component and the health, including the "recommendations related to the reduction of a disease risk" linked to the significant reduction of a risk factor in the development of a human disease.

In the present invention, the expressions "healthy recommendation/claim", "healthy recommendations/claims" or "recommendations/claims for the health" are intended to refer to the recommendations/claims established by the Regulation 1924/2006, which concern the existence of a relationship between a food (or a component thereof) and human health.

The process of the invention is applicable to all virgin vegetable oils, i.e., extracted exclusively by mechanical means and not subjected to chemical refining processes that deplete the phenolic component of hydrophilic nature.

In the present invention, the oil is a virgin vegetable oil and the expression "oil sample" means a sample of virgin vegetable oil.

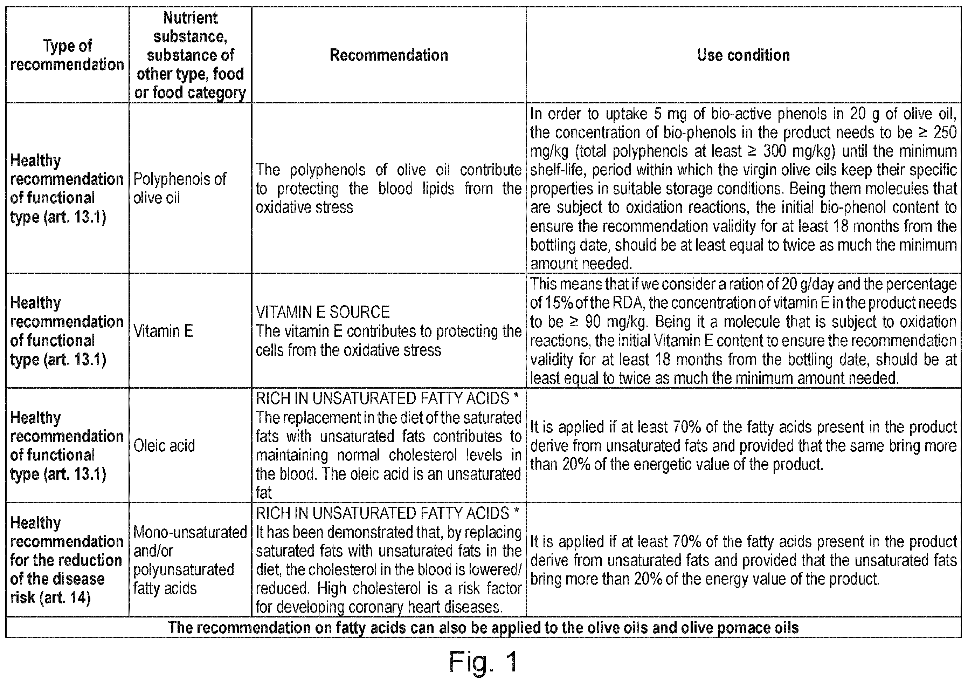

Advantageously, the process object of the present invention allows to evaluate and predict the validity of the existence of a relationship between the polyphenols contained in a virgin vegetable oil, preferably in the extra-virgin olive oil, and human health. The approved healthy recommendations for the virgin vegetable oil are related to the content of polyphenols, vitamin E, oleic acid and mono-unsaturated and/or polyunsaturated fatty acids and are depicted in Figure 1. In Figure 1 it is possible to read the wording "healthy recommendation of functional type". The expression "healthy recommendation of functional type" refers to the recommendations which attribute to the food product (e.g. oil) a function directed to improve some aspects of human health and therefore are healthy recommendations. Such healthy recommendations/claims of functional type are defined in the Article 13 of the Regulation 1924/2006 and describe particular health benefits other than reducing the risk of disease (e.g. aiding digestion, strengthening bones, etc.). In Figure 1 it is also possible to read the wording "healthy recommendation/claim for the reduction of the disease risk". The healthy recommendation/claim for the reduction of the disease risk is defined in the Article 14 of the Regulation 1924/2006. The present invention relates, in particular, to the healthy recommendation related to the polyphenols in extra-virgin olive oil. The polyphenols are a class of compounds that give the oil stability, nutritional and healthy qualities as well as sensory peculiarities. They contribute to the protection of blood lipids from the oxidative stress.

As it is possible to be seen under the heading "polyphenols of the olive oil" in Figure 1, in order for 5 mg of bioactive phenols to be taken in 20 g of olive oil, the concentration of bio-phenols in the product must be greater than or equal to 250 mg/kg up to the minimum shelf-life, the period within which virgin olive oils maintain their specific properties under appropriate storage conditions.

By the terms "bio-phenols" or "bioactive phenols" is meant to refer to biologically active phenols, i.e., hydroxytyrosol and derivatives thereof. Hydroxytyrosol is a plant chemical compound present in the olive oil in the form of its ester with elenolic acid (oleuropein). It is known for its strong antioxidant properties.

By the terms "total polyphenols" or "total phenols" is meant to refer to the total amount of phenols, comprising both biologically active phenols (80%), i.e., bio-phenols as defined above, and non-biologically active phenols (20%).

The process according to the invention allows to determine the minimum shelf-life of the virgin vegetable oil, more preferably of the healthy extra-virgin olive oil. Said minimum shelf-life coincides with the period of validity of the healthy recommendation, i.e. the healthy recommendations related to the polyphenols present in the oil sample under investigation. Said minimum shelf-life of the oil sample and said period of validity of the healthy recommendation related to the polyphenols of an oil sample is determined from the level of polyphenols detected in the oil sample under investigation and processed on the basis of a predictive model.

The process according to the invention provides for performing a colorimetric chemical assay based on the oxidation of the polyphenols present in said oil sample into o-quinones, with production of a chromophore which can be quantified colorimetrically (step a) of the process). According to the invention, said colorimetric chemical assay is carried out on filter paper, for example Whatman filter paper. In an embodiment of the process, NaIO₄ and MBTH are immobilized on said filter paper. Sodium meta-periodate (NaIO₄) is the oxidizing agent which promotes the oxidation of the phenols present in the olive oil sample under investigation, into the corresponding o-quinones, by consuming molecular oxygen. Said o-quinones react with 3-methyl-2-benzothiazolinone hydrazone (MBTH) to generate a pink chromophore compound (CGA-q-MBTH) which can be quantified colorimetrically. In another embodiment, the process of the invention comprises, upstream of step a), a step a') of inserting the oil sample into pre-dosed tubes comprising a mixture of ethyl ether/ethyl alcohol and water and a step a") of stirring said mixture. At this point, the contents of the tube (comprising the mixture of oil, ethyl ether/ethyl alcohol and water) are poured onto filter paper on which NaIO₄ and MBTH are immobilized.

In another embodiment of the process of the invention, the filter paper used for the chemical assay according to step a) of the process, does not comprise NaIO₄ and MTBH. In this further embodiment, NaIO₄ and MTBH may be placed in the same tube in which the oil sample will be placed. The contents of said tube shall then be poured onto the filter paper.

The levels of polyphenols present in the sample are detected based on the staining intensity of the pink chromophore compound (CGA-q-MBTH) that is created by the reaction of the o-quinones (generated by the oxidation of the phenols in the oil sample) with MBTH. Therefore, the colorimetric chemical assay generates quantitative color data, i.e. indicative of the quantity of the polyphenol level in the oil sample under investigation. According to the process of the invention, the readout of the intensity of the color data is carried out by means of the camera of an electronic device (step b) of the process). In a particularly preferred embodiment, the step b) of the process, i.e., the readout of the intensity of said color data derived from the chemical assay, is carried out by using a camera of a smartphone, computer or tablet. In a particularly preferred embodiment, the electronic device according to step b) of the process is a smartphone. Said smartphone uses the camera to automatically carry out a colorimetric analysis thanks to an app capable of quantifying the amount of phenols of the sample with high precision and reproducibility. The app incorporates a predictive model developed by the Applicant based on experiments carried out in the laboratory and based on the realization of a calibration line. Said calibration line was obtained according to the experiments described in Example 1.

The color data detected through the camera of an electronic device, more preferably a smartphone, is processed thanks to a predictive model developed by the Applicant.

Said predictive model is based on an algorithm that processes the color information by displaying the corresponding value in terms of shelf-life on the screen of the smartphone used for the readout of the color intensity of the pink chromophore compound developed by the chemical assay according to step a) of the process of the invention.

In a preferred embodiment, said predictive model developed by the Applicant is incorporated into a data processor, for example an app.

After having photographed the pink chromatic compound, intensity data related to the color intensity of the compound are obtained (thanks to a calibration line that allows to obtain an intensity value) and parameters related to chemical, physical, biochemical and physical-chemical properties of the oil, packaging and environmental conditions are uploaded in said app (in which the predictive model is incorporated).

At this point, the predictive model incorporated in an app and based on the above algorithm, integrates this information with the color intensity of the chromatic compound to return a value on the minimum shelf-life of the oil.

Advantageously, said predictive model is able to determine the validity of the healthy recommendations related to the polyphenols present in a sample of virgin vegetable oil and to determine the minimum term of validity of the healthy recommendation of said oil sample.

The predictive model used in step c) of the process of the invention is based on an algorithm that processes the color information by displaying the corresponding value in terms of shelf-life on the screen of the smartphone used for the readout of the color intensity of the pink chromophore compound developed by the chemical assay according to step a) of the process of the invention.

By the term "shelf-life" is meant to denote the shelf-life, i.e. the period of time during which goods can be stored without becoming unsuitable for use, consumption or sale and without the recommendations on the label becoming invalid. An oil may after a certain period lose its polyphenol content for the application of the healthy recommendation, but it can still be classified as extra-virgin oil and suitable for human consumption. However, if there is an invalid healthy recommendation on the label, the manufacturer is subject to penalties for trade fraud. In the study of commodities, shelf-life takes the meaning of: "period between production and sale during which the total quality of the product must be preserved unchanged", including the validity of a healthy recommendation printed on the label. Said predictive model used in step c) of the process of the invention was developed by the Applicant which took into account variables incorporated in the calibration lines and predictive models developed by technological preservation trials (cultivar, harvesting time, extraction system, packaging size and material, inertization, sales destination and trade routes).

The predictive model used in the process of the invention simultaneously incorporates:
i) intrinsic characteristics of the product (i.e. the oil);
ii) packaging properties: migration and barrier capacity of the material used for packaging; and
iii) extrinsic variables influencing the quality decay of the product.

The characteristics i) and iii) which the predictive model of the process of the invention takes into account refer to the intrinsic characteristics of the oil under investigation and to the extrinsic variables affecting the quality decay of said oil under investigation. Therefore, by the term "product", with reference to the characteristics i) and iii), is meant olive oil, preferably extra-virgin olive oil.

According to the invention, said intrinsic characteristics of the product i) are the chemical, physical, biochemical and physical-chemical properties of the product itself, depending on the technological operations applied during the production process. According to the invention, said packaging properties ii) relate to the migrations and barrier capacity of the material used for packaging the product (moisture, oxygen or other gases, light and heat). By "migration" is meant the transfer of matter from the food to the packaging material and vice versa. Ideally, a material contacting food should not result in transfer of matter to the food itself (via positive migration) and/or from the food to the material (via negative migration).

According to the invention, the extrinsic variables influencing the quality decay of the product iii) which the predictive model of the process of the invention takes into account, are for example the environmental conditions. Included in the category of extrinsic variables iii) that influence the quality decay of the product, i.e. the oil, are all the variables that affect the quality of the product during the distribution, retail, up to purchase, storage and handling by the consumer. The characteristics i), ii) and iii) described above are evaluated by the predictive model of the process according to the invention in order to return in real time a value of the minimum shelf-life of the oil under investigation and to define the applicability and validity of the healthy recommendations related to the polyphenols. In other words, the process of the invention allows to detect the level of polyphenols in an oil sample and to return in real time a value of the minimum shelf-life of the oil under investigation.

The healthy recommendations related to the polyphenols in an oil sample are temporally valid when the level of bio-phenols in 20 g of said sample is at least greater than or equal to 5 mg (hydroxytyrosol and derivatives thereof), which correspond to 250 mg of bio-phenols in 1 kg of oil, preferably greater than or equal to 300 mg total polyphenols in 1 kg of oil (of which 80% are bio-phenols).

The process of the invention allow to identify a minimum shelf-life, i.e. the date until which the food product retains its specific properties under appropriate storage conditions, when the level of bio-phenols in 20 g of said oil sample under investigation is greater than or equal to 5 mg, when the level of bio-phenols in 1 kg of oil is greater than or equal to 250 mg of bio-phenols, even more preferably when the level of total polyphenols in 1 kg of oil is greater than or equal to 300 mg. According to the invention, the period of validity of the healthy recommendations and the minimum shelf-life coincide. Furthermore, the process of the invention allows the applicability and validity of the healthy recommendations related to the polyphenols to be defined on the basis of parameters that take into account the intrinsic characteristics of the oil, the packaging properties and the environmental conditions. Therefore, said process is very useful in order to define marketing strategies for all those involved in the olive oil field. The process of the invention does not require complex equipment and, therefore, is a low cost process. Furthermore, information of particular importance to those involved in the olive oil field can be obtained within a short period of time. In particular, the data processing carried out thanks to step c) of the process takes the form of a system that relieves the operator in the olive oil field of the need to take decisions on the applicability and validity of the healthy recommendations related to the polyphenols. Another advantage results from the fact that the process of the invention does not require specialized personnel and that the access to the test results is complete, easy and secure. In fact, by exploiting the connectivity of the smartphone, it is possible to get immediate access via "cloud" to the results of tests carried out on samples of olive oil. The "cloud" is a personal storage space that can be accessed anytime, anywhere simply by using any Internet connection.

A further object of the invention is an apparatus for evaluating the temporal validity of the healthy recommendations related to the polyphenols present in an oil sample, comprising
- a first chamber, including a filter paper,
- a scanning chamber comprising an electronic device fitted with a camera and
- a data processor configured to carry out step c) of the process according to the invention, i.e. configured to process the color data derived from the chemical assay on filter paper carried out in the first chamber of the apparatus, by means of a predictive model adapted to determine the minimum shelf-life of said oil sample.

In a preferred embodiment, the filter paper in the first chamber of the apparatus comprises NaIO₄ and MBTH immobilized on its surface, which are necessary to carry out the colorimetric chemical assay on which step a) of the process of the invention is based. Said filter paper may for example be a Whatman filter paper. In the first chamber the colorimetric chemical assay takes place with production of the pink chromophore compound (CGA-q-MBTH). The first chamber may be connected to the scanning chamber of the apparatus, through a connecting structure. Through the connecting structure, the sample characterized by the presence of the pink chromophore compound (CGA-q-MBTH) is brought to the scanning chamber, where the color intensity of said chromophore is read and data processing takes place. The connecting structure of the apparatus of the invention may be, for example, a movable structure allowing the movement of the sample, characterized by the presence of the pink chromophore compound (CGA-q-MBTH), from the first chamber of the apparatus to the scanning chamber of said apparatus. In the scanning chamber there is an electronic device provided with a camera and a data processor based on a predictive model, capable of determining the minimum shelf-life of said sample, in particular an oil sample, according to the present invention. In a preferred embodiment, said electronic device provided with a camera is selected from smartphone, tablet or computer. In a particularly preferred embodiment, said electronic device provided with a camera is a smartphone. The data processor present in the apparatus of the invention may be included in said electronic device provided with a camera. According to a particularly preferred embodiment, said data processor is an app included in a smartphone. The data processor present in the apparatus of the invention, for example an app included in a smartphone, comprises an algorithm based on a predictive model that processes the color data derived from the chemical assay carried out in the first chamber of the apparatus and returns a shelf-life value on the screen of the mobile phone.

Said predictive model has been developed by the present patent application is based on variables integrated in the calibration curves of the various experiments carried out (Example 1) and on predictive models developed by technological preservation trials. Said predictive model, embedded in a smartphone app, simultaneously takes into account the chemical, physical, biochemical and physical-chemical properties of the oil sample, which depend on the technological operations applied during the production process, the environmental conditions in which the oil is stored and the packaging materials of the oil.

In other words, the apparatus of the invention allows to predict a shelf-life of an oil sample and to define a time limit of validity of the healthy recommendations related to the polyphenols present in said sample, based on the level of said polyphenols detected therein and based on other parameters which take into account the intrinsic characteristics of the product (i.e., the oil sample), the properties of the packaging, such as the type of material used for packaging, and the extrinsic variables which influence the qualitative decay of the product, such as for example the environmental conditions. In a preferred embodiment, the apparatus of the invention works with reaction tubes containing liquid and stable reagents already dosed for the reaction, in particular a mixture of ethyl ether/ethyl alcohol and water. The only task of the operator is to pipette the sample into said tube in a quantity predetermined by the capacity of the pipette and stir vigorously, as all the analytical phases take place automatically. After the agitation is completed, the contents of the pipette will be poured into the filter paper in the first chamber of the apparatus according to the invention. After about 15 minutes, once the colorimetric chemical reaction has taken place, the intensity of the color will be read directly with the camera of the smartphone, by entering the technological parameters whose variables are an integral part of the algorithm that will be able to return in real time the validity of the healthy recommendations related to the polyphenols and the minimum shelf-life of the oil under investigation. To date, there is no instrument capable of combining rapid and low cost chemical assays with smart-phone technology and offering the operators in the olive oil field a tool that can be used immediately to define marketing strategies. Said apparatus is able to increase the potential marketing and profit opportunities of the oil producers, since it is able to determine the validity of the healthy recommendations related to the polyphenols contained in the olive oil and to define the minimum shelf-life of said oil, thanks to the predictive model incorporated in it.

Said apparatus is able to increase the potential opportunities of logistic management of the oil mill since it allows the storage of the product for homogeneous batches in phenolic composition by protecting the high quality product from mixing (ordinary procedure of the oil mills) with products of lower quality.

Furthermore, an object of the invention is a kit for evaluating the temporal validity of the healthy recommendations related to the polyphenols present in an oil sample by means of the above described process, comprising at least one pre-dosed tube comprising a mixture of ethyl ether/ethyl alcohol and water, at least one filter paper, at least one electronic device provided with a camera and at least one data processor allowing to determine the minimum shelf-life of the oil under investigation, thus defining the applicability and validity of the healthy recommendations related to the polyphenols. According to a preferred embodiment, the filter paper comprises NaIO₄ and MBTH immobilized thereon. The data processor comprised in the kit of the invention, is based on a predictive model constituted by an algorithm allowing to determine the minimum shelf-life of the oil under investigation and to define the applicability and validity of the healthy recommendations related to the polyphenols. According to a particularly preferred embodiment, said electronic device is a smartphone and said data processor is included in the electronic device and is an app. Therefore, preferably, the kit of the invention comprises at least one smartphone including an app allowing to determine the minimum shelf-life of the oil under investigation and to define the applicability and validity of the healthy recommendations related to the polyphenols contained in said oil.

### BRIEF DESCRIPTION OF THE FIGURES

- Figure 1: is a list of the authorized healthy recommendations for the olive oil.
- Figure 2: depicts the steps of the process according to the invention.
- Figure 3: is a schematic representation of the apparatus of the invention.

### DESCRIPTION OF THE FIGURES

In Figure 1 the healthy recommendations/claims authorized by the Regulation for the olive oil are set out. In Figure 1, said healthy recommendations are classified based on the type of recommendation (healthy recommendation of functional type and healthy recommendation for the reduction of the disease risk), the substance on which the healthy recommendation is based (olive oil polyphenols, vitamin E, oleic acid, mono-unsaturated and/or polyunsaturated fatty acids), the healthy recommendation and the condition of use. The healthy recommendation on which the present invention is based is that related to the olive oil polyphenols.

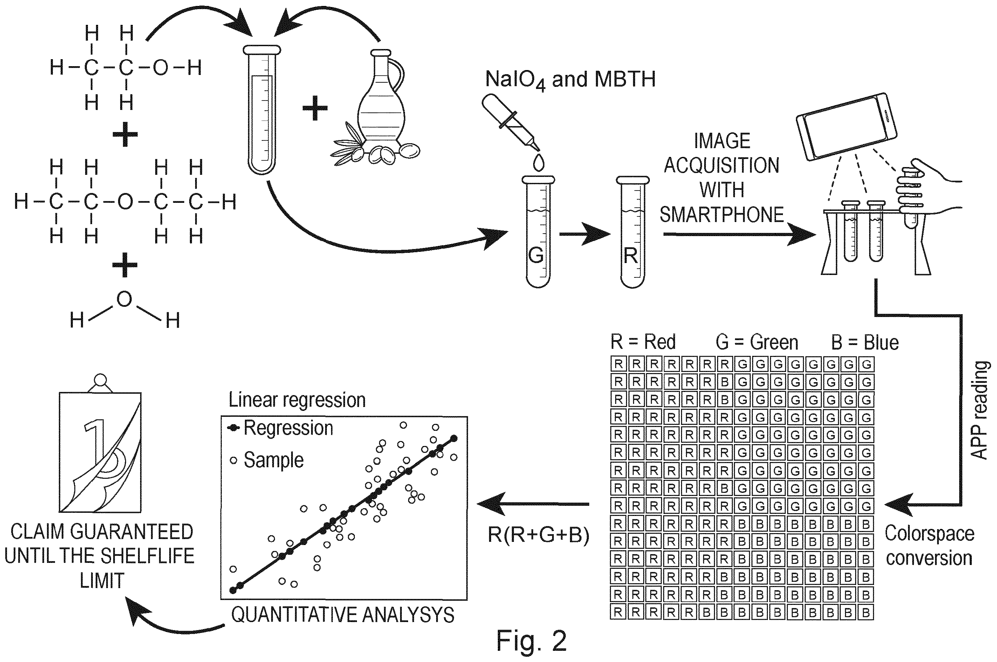

Figure 2 depicts the process steps of the invention. On the left of Figure 2, the step a') of inserting the oil sample under investigation into pre-dosed tubes comprising a mixture of ethyl ether/ethyl alcohol and water and obtaining a mixture comprising oil, ethyl ether/ethyl alcohol and water is depicted. At this point NaIO₄ and MTBH can be placed in the tubes comprising the mixture of oil, ethyl ether/ethyl alcohol and water. The contents of the tube are then poured onto a filter paper.

In an alternative embodiment, a paper specimen may be immersed in said tube comprising the mixture of oil, ethyl ether/ethyl alcohol and water. The readout of the color intensity of the chromophore developed by the reaction is carried out by an electronic device in which the predictive model developed by the Applicant is incorporated. With reference to Figure 2, the readout of the color intensity of the chromophore that develops from the reaction, the color conversion and the quantitative analysis is carried out via a smartphone app.

By means of free apps, the camera of a smartphone allows to capture RGB values in real time. At this point, there is the quantitative analysis by means of an algorithm developed by the Applicant and which allows to achieve a value in terms of shelf-life. This shelf-life coincides with the minimum term of validity of the healthy recommendations related to the polyphenols in the olive oil. The steps of the process are depicted in Figure 2 by an arrow and legend.

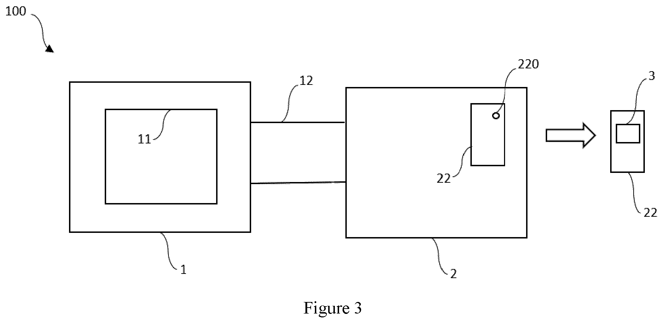

Figure 3 depicts an apparatus 100 for evaluating the temporal validity of the healthy recommendations related to the polyphenols present in an olive oil sample. Said apparatus 100 comprises a first chamber 1 in which there is a filter paper 11, a scanning chamber 2 comprising an electronic device 22 provided with a camera 220 and a data processor 3 configured to carry out the step c) of the process according to claim 1. In particular, the filter paper 11 in the first chamber 1 has the compounds NaIO₄ and MBTH immobilized on its surface, which are necessary to carry out the colorimetric chemical assay on which the step a) of the process of the invention is based. In the first chamber 1 the colorimetric chemical assay takes place with the formation of the pink chromophore compound (CGA-q-MBTH). The first chamber 1 may be connected to the scanning chamber 2 through a connecting structure 12. In the scanning chamber 2, there is an electronic device 22 provided with a camera 220 and a data processor 3 based on a predictive model capable of determining the minimum shelf-life of said oil sample. Said data processor may be included in said electronic device 22 equipped with a camera 220. In fact, in a particularly preferred embodiment, the electronic device 22 provided with a camera 220 is a smartphone and the data processor 3 is an app included in said smartphone. The app included in said smartphone processes the color data according to step b) of the process by means of a predictive model integrating i) the intrinsic characteristics of the product (i.e., the oil under investigation), preferably at least one among the chemical, physical, biochemical and physical-chemical properties of said product, ii) the packaging properties, preferably the type of material used for packaging, and iii) the extrinsic variables affecting the quality decay of the product, preferably the environmental conditions.

### EXPERIMENTAL SECTION

### Example 1

### Measurement of the polyphenol level via the Folin Ciocalteau process and determination of the color intensity via benchtop colorimeter

Starting from an oil with a concentration of total polyphenols equal to 1000 mg/kg (evaluated by the Folin process), dilutions were prepared with rectified olive oil (total polyphenols equal to 0 mg/kg) according to what depicted in Table 1 (identical composition in fatty acids but free of polyphenols - total polyphenols equal to 0 mg/kg).

**Table 1**

| **Sample** | **% extra virgin olive oil 1000 ppm** | **% rectified olive oil** |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |
| 7 | 40 | 60 |
| 8 | 30 | 70 |
| 9 | 20 | 80 |
| 10 | 10 | 90 |
| 11 | 0 | 100 |

The concentration of polyphenols for each sample was determined by the Folin Ciocalteau process. Before the reaction with the mixture of phosphotungstic and phosphomolybdic acid, which in the presence of polyphenols oxidizes and generates a colored complex which absorbs at 765 nm, the sample was prepared according to the following steps:
1) About 10 g of oil is weighed in a beaker with mg accuracy.
2) 10 ml of 80:20 methanol-water mixture is added to extract the polyphenols from the oil.
3) Stirring for 30 minutes, waiting for 15 minutes and centrifuging.
4) 2 phases are formed. Taking the upper phase and transferring in a 25 ml volumetric flask.
5) 10 ml of 80:20 methanol-water is added again to the remaining oil,
6) Stirring, centrifuging and separating the upper phase by transferring it in the same 25 ml flask;
7) Making up to the mark with the methanol/water mixture.
8) The methanol solution is frozen, melted and filtered to remove any possible traces of oil.

To determine the concentration of polyphenols, a calibration line was used, obtained by preparing a stock solution of gallic acid at the concentration of 1000 ppm of 80:20 methanol-water and obtaining samples at 100, 250, 500, 750 ppm by dilution from this solution. For each dilution of the standard the colorimetric test is performed and, after evaluating the absorbance at 765 nm, the calibration line is constructed. The same samples, once verified the content in total polyphenols, were used for the reaction with sodium meta-periodate (NaIO₄) immobilized on filter paper according to step a) of the process, thus obtaining a pink chromophore which can be quantified colorimetrically. A first check correlating the intensity of the pink color with the content of total phenols measured by the Folin Ciocalteau process was carried out with a benchtop colorimeter according to the following method:
1. Solubilizing 1 g of extra-virgin olive oil in a 1:2 binary mixture of ethyl ether and ethyl alcohol (10 ml) in a disposable vial equipped with a stopper;
2. Vortexing for 30 seconds;
3. Adding 1 ml of a saturated solution (with the presence of salt sediment) of NaIO₄ in distilled water;
4. Vortexing for 30 seconds;
5. Readout of the color intensity of the chromophore compound CGA-q-MBTH by benchtop colorimeter.

The oxidation of the polyphenols in excess of NaIO₄ is an instantaneous reaction and generates a pink chromophore whose intensity is related to the concentration of polyphenols. The intensity of the pink chromophore was preliminarily measured with a benchtop colorimeter for liquid solutions. The finding of a positive correlation was followed by the next experimental step.

### Example 2

### Carrying out the process of the invention

NaIO₄ and MBTH were dissolved and diluted in phosphate buffer at pH 6.5. The buffer was prepared with KH₂PO₄ and NaOH. Whatman filter paper (CAT No. 1095.093) was used as the backing material. The paper was cut into 1 × 4 cm² and the filter paper specimens were immersed in a solution of NaIO₄ (10 mM) and MBTH (25 mM) in 1.5:1 ratio (v/v).

Said paper specimens were dried at room temperature under fume hood for 1 hour and 1 g of the olive oil samples depicted in Table 1 were dissolved in a 1:2 mixture of ethyl ether and ethyl alcohol (10 ml) according to step a') of the process of the invention. At this point, the paper specimens were immersed in the oil samples dissolved in the binary mixture of ethyl ether and ethyl alcohol for the reaction to take place between the polyphenols present in the oil sample and NaIO₄ and MBTH on the filter paper (step a) of the process of the invention). Color measurements were then analyzed by scanning the specimens 15 minutes after the reaction initiation with ImageJ software for Windows by using RGB coordinates. The results were used to test the correlations with:
- the content of total polyphenols measured with oil samples (mixtures depicted in Table 1) by the Folin Ciocalteau process (Example 1); and
- the color intensity values measured for the same samples with the NaIO₄-MBTH colorimetric process and the benchtop colorimeter (Example 1).

Subsequently, the same scanned specimens were analyzed with the smartphone camera by means of free apps (iOS App: Color Assist Free Edition; App Android: Color Grab free) using the iPhone or iPad camera to capture RGB values in real time (step b) of the process of the invention). The data were transferred to Excel and transformed through the application of an algorithm resulting from shelf-life studies into a time value (months), the limit beyond which the concentration of bio-phenols of the product falls below 250 mg/kg and the healthy recommendation will no longer be valid.

Such time value can be used to set forth the minimum shelf-life on the bottles bearing the healthy recommendation related to the polyphenols in the oil, i.e. the date until which the food product retains its healthy properties under appropriate storage conditions.

## Claims

1. A process for evaluating the time validity of the healthy recommendations related to the polyphenols present in a sample of virgin vegetable oil, comprising the steps of:
a) obtaining color data of the polyphenol level in said sample of virgin vegetable oil by colorimetric chemical assay, said color data being quantitative data of the level of said polyphenols in said oil sample;
b) reading the intensity of said color data of step a) by an electronic device provided with a camera; and **characterized by**
c) processing the data of step b) by a predictive model adapted to determine the minimum term of validity of the healthy recommendation of said oil sample wherein the predictive model simultaneously incorporates the intrinsic characteristics of the oil, properties of the packaging; and extrinsic variables which influence the qualitative decay of the product,
said colorimetric chemical assay is based on the oxidation of polyphenols present in said oil sample into o-quinones, producing a chromophore which is colourimetrically quantified, said process using sodium meta-periodate (NaIO4) acting as the oxidizing agent by promoting the oxidation of the polyphenols into the corresponding o-quinones, said o-quinones reacting with 3-methyl-2-benzothiazolinone hydrazone (MBTH) and generating a pink chromophore compound (CGA-q-MBTH) which can be quantified colorimetrically.

2. The process according to claim 1, wherein said colorimetric chemical assay according to step a) is carried out on filter paper on which said reagents sodium-meta-periodate (NaIO₄) and 3-methyl-2-benzothiazolinone hydrazone (MBTH) are immobilized.

3. The process according to claim 1, comprising upstream of step a), a step a') of inserting the oil sample into pre-dosed tubes comprising a mixture of ethyl ether/ethyl alcohol and water, obtaining a mixture comprising oil, ethyl ether/ethyl alcohol and water and a step a") of stirring said mixture.

4. The process according to claim 1, wherein said virgin vegetable oil is extra-virgin olive oil.

5. The process according to claim 1, wherein reading the color intensity according to step b) of the process is carried out by a camera of an electronic device selected from smartphone, tablet or computer, more preferably smartphone.

6. The process according to claim 1, **characterized in that** said predictive model according to step c) of the process is integrated in said electronic device provided with a camera according to step b) of the process.

7. The process according to claim 1, **characterized in that** the predictive model according to step c) simultaneously integrates i) intrinsic characteristics of the product, such as the chemical, physical, biochemical and physical-chemical properties, ii) packaging properties, such as the type of material used for the packaging, and iii) extrinsic variables which influence the qualitative decay of the product, such as the environmental conditions.

8. The process according to claim 1, **characterized in that** a bio-phenol level at least greater than or equal to 250 mg/kg of said oil sample, preferably greater than or equal to 300 mg/kg of total polyphenols, denotes the validity of the healthy recommendations related to the polyphenols in said oil sample and said minimum shelf-life according to step c) of the process is the date until which the oil sample has a bio-phenol level in 20 g of oil at least greater than or equal to 5 mg.

9. An apparatus (100) adapted to perform the process of claim 1 for evaluating the time validity of the healthy recommendations related to the polyphenols present in a sample of virgin vegetable oil, **characterized in that** it comprises a first chamber (1) comprising a filter paper (11) wherein the filter paper (11) has either (A) the compounds sodium meta-periodate (NaIO₄) and 3-methyl-2-benzothiazolinone hydrazone (MBTH) immobilized on its surface or (B) when the filter paper does not comprise the compounds immobilized on its surface the compounds sodium meta-periodate (NaIO₄) and 3-methyl-2-benzothiazolinone hydrazone (MBTH) are added separately into a pre-dosed tube comprising a mixture of ethyl ether/ethyl alcohol and water, the apparatus further comprising:
a scanning chamber (2) comprising an electronic device (22) provided with a camera (220) selected from a smartphone or tablet and wherein said electronic device (22) further comprises a data processor (3) configured to carry out step c) of the process of claim 1.

## Patentansprüche

1. Ein Verfahren zum Auswerten der zeitlichen Gültigkeit von Gesundheitsempfehlungen in Bezug auf die in einer Probe von nativem pflanzlichem Öl vorhandenen Polyphenole, umfassend die Schritte:
a) Erhalten von Farbdaten des Polyphenolgehalts in der Probe von nativem pflanzlichem Öl mittels einer kolorimetrischen chemischen Analyse, wobei die Farbdaten quantitative Daten über den Gehalt der Polyphenole in der Ölprobe sind;
b) Auslesen der Intensität der Farbdaten aus Schritt a) durch ein elektronisches Gerät, das mit einer Kamera versehen ist; und **gekennzeichnet durch**
c) Verarbeiten der Daten aus Schritt b) mittels eines prädiktiven Modells, das angepasst ist, um die minimale Gültigkeitsdauer der Gesundheitsempfehlung der Ölprobe zu bestimmen, wobei das prädiktive Modell gleichzeitig die intrinsischen Eigenschaften des Öls, die Eigenschaften der Verpackung und extrinsische Variablen, welche den qualitativen Abbau des Produkts beeinflussen, integriert,
wobei die kolorimetrische chemische Analyse auf der Oxidation der in der Ölprobe vorhandenen Polyphenole zu o-Chinonen basiert, wodurch ein Chromophor gebildet wird, das kolorimetrisch quantifiziert wird, wobei das Verfahren Natriummetaperiodat (NaIO₄) als Oxidationsmittel verwendet, das die Oxidation der Polyphenole zu den entsprechenden o-Chinonen fördert, wobei die o-Chinone mit 3-Methyl-2-benzothiazolinonhydrazon (MBTH) reagieren und eine rosa Chromophorverbindung (CGA-q-MBTH) erzeugen, die kolorimetrisch quantifiziert werden kann.

2. Das Verfahren nach Anspruch 1, wobei die kolorimetrische chemische Analyse gemäß Schritt a) auf Filterpapier durchgeführt wird, auf dem die Reagenzien Natriummetaperiodat (NaIO₄) und 3-Methyl-2-benzothiazolinonhydrazon (MBTH) immobilisiert sind.

3. Das Verfahren nach Anspruch 1, umfassend, stromaufwärts von Schritt a), einen Schritt a'), bei dem die Ölprobe in vordosierte Röhrchen eingebracht wird, die ein Gemisch aus Ethyläther/Ethylalkohol und Wasser enthalten, wodurch ein Gemisch aus Öl, Ethyläther/Ethylalkohol und Wasser erhalten wird, und einen Schritt a") des Rührens des Gemischs.

4. Das Verfahren nach Anspruch 1, wobei das native pflanzliche Öl natives Olivenöl extra ist.

5. Das Verfahren nach Anspruch 1, wobei das Auslesen der Farbintensität gemäß Schritt b) des Verfahrens mittels einer Kamera eines elektronischen Geräts ausgewählt aus Smartphone, Tablet oder Computer, vorzugsweise Smartphone, durchgeführt wird.

6. Das Verfahren nach Anspruch 1, **dadurch gekennzeichnet, dass** das prädiktive Modell gemäß Schritt c) des Verfahrens in das genannte elektronische Gerät mit einer Kamera gemäß Schritt b) des Verfahrens integriert ist.

7. Das Verfahren nach Anspruch 1, **dadurch gekennzeichnet, dass** das prädiktive Modell gemäß Schritt c) gleichzeitig integriert: i) intrinsische Eigenschaften des Produkts wie chemische, physikalische, biochemische und physikalischchemische Eigenschaften, ii) Eigenschaften der Verpackung wie die Art des für die Verpackung verwendeten Materials, und iii) extrinsische Variablen, welche den qualitativen Abbau des Produkts beeinflussen, wie die Umweltbedingungen.

8. Das Verfahren nach Anspruch 1, **dadurch gekennzeichnet, dass** ein Biophenolgehalt von mindestens größer oder gleich 250 mg/kg der Ölprobe, vorzugsweise größer oder gleich 300 mg/kg Gesamtpolyphenole, die Gültigkeit der Gesundheitsempfehlungen in Bezug auf die Polyphenole in der Ölprobe anzeigt und dass die minimale Haltbarkeitsdauer gemäß Schritt c) des Verfahrens das Datum ist, bis zu dem die Ölprobe in 20 g Öl einen Biophenolgehalt von mindestens größer oder gleich 5 mg aufweist.

9. Eine Vorrichtung (100), angepasst zur Durchführung des Verfahrens nach Anspruch 1 zum Auswerten der zeitlichen Gültigkeit der Gesundheitsempfehlungen in Bezug auf die in einer Probe von nativem pflanzlichem Öl vorhandenen Polyphenole, **dadurch gekennzeichnet, dass** sie eine erste Kammer (1) umfasst, die ein Filterpapier (11) umfasst, wobei das Filterpapier (11) entweder (A) die Verbindungen Natriummetaperiodat (NaIO₄) und 3-Methyl-2-benzothiazolinonhydrazon (MBTH) auf seiner Oberfläche immobilisiert hat oder (B), wenn das Filterpapier die Verbindungen nicht auf seiner Oberfläche immobilisiert enthält, die Verbindungen Natriummetaperiodat (NaIO₄) und 3-Methyl-2-benzothiazolinonhydrazon (MBTH) getrennt in ein vordosiertes Röhrchen eingebracht werden, das ein Gemisch aus Ethyläther/Ethylalkohol und Wasser umfasst, wobei die Vorrichtung ferner umfasst:
eine Scankammer (2) mit einem elektronischen Gerät (22), das mit einer Kamera (220) versehen ist, ausgewählt aus einem Smartphone oder Tablet, und wobei das elektronische Gerät (22) ferner einen Datenprozessor (3) umfasst, der zur Durchführung von Schritt c) des Verfahrens nach Anspruch 1 konfiguriert ist.

## Revendications

1. Procédé d'évaluation de la validité temporelle des recommandations sanitaires relatives aux polyphénols présents dans un échantillon d'huile végétale vierge, comprenant les étapes consistant à :
a) obtenir des données de couleur du niveau de polyphénols dans ledit échantillon d'huile végétale vierge par essai chimique colorimétrique, lesdites données de couleur étant des données quantitatives du niveau desdits polyphénols dans ledit échantillon d'huile ;
b) lire l'intensité desdites données de couleur de l'étape a) à l'aide d'un dispositif électronique muni d'une caméra ; et **caractérisé par**
c) traiter les données de l'étape b) par un modèle prédictif adapté pour déterminer la durée minimale de validité de la recommandation santé dudit échantillon d'huile, dans lequel le modèle prédictif intègre simultanément les caractéristiques intrinsèques de l'huile, les propriétés de l'emballage ; et les variables extrinsèques qui influencent la détérioration qualitative du produit,
ledit essai chimique colorimétrique est basé sur l'oxydation des polyphénols présents dans ledit échantillon d'huile en o-quinones, produisant un chromophore qui est quantifié par colorimétrie, ledit procédé utilisant du métaperiodate de sodium (NaIO4) agissant comme agent oxydant en favorisant l'oxydation des polyphénols en o-quinones correspondantes, lesdites o-quinones réagissant avec la 3-méthyl-2-benzothiazolinone hydrazone (MBTH) et générant un composé chromophore rose (CGA-q-MBTH) qui peut être quantifié par colorimétrie.

2. Procédé selon la revendication 1, dans lequel ledit essai chimique colorimétrique selon l'étape a) est réalisé sur du papier filtre sur lequel lesdits réactifs, le métaperiodate de sodium (NaIO₄) et la 3-méthyl-2-benzothiazolinone hydrazone (MBTH), sont immobilisés.

3. Procédé selon la revendication 1, comprenant en amont de l'étape a), une étape a') consistant à insérer l'échantillon d'huile dans des tubes prédosés comprenant un mélange d'éther éthylique/alcool éthylique et d'eau, à obtenir un mélange comprenant de l'huile, de l'éther éthylique/alcool éthylique et de l'eau, et une étape a") consistant à agiter ledit mélange.

4. Procédé selon la revendication 1, dans lequel ladite huile végétale vierge est de l'huile d'olive extra vierge.

5. Procédé selon la revendication 1, dans lequel la lecture de l'intensité de couleur selon l'étape b) du procédé est effectuée par une caméra d'un dispositif électronique choisi parmi un smartphone, une tablette ou un ordinateur, de préférence un smartphone.

6. Procédé selon la revendication 1, **caractérisé en ce que** ledit modèle prédictif selon l'étape c) du procédé est intégré dans ledit dispositif électronique muni d'une caméra selon l'étape b) du procédé.

7. Procédé selon la revendication 1, **caractérisé en ce que** le modèle prédictif selon l'étape c) intègre simultanément i) les caractéristiques intrinsèques du produit, telles que les propriétés chimiques, physiques, biochimiques et physico-chimiques, ii) les propriétés d'emballage, telles que le type de matériau utilisé pour l'emballage, et iii) les variables extrinsèques qui influencent la détérioration qualitative du produit, telles que les conditions environnementales.

8. Procédé selon la revendication 1, **caractérisé en ce qu'**un niveau de bio-phénols au moins supérieur ou égal à 250 mg/kg dudit échantillon d'huile, de préférence supérieur ou égal à 300 mg/kg de polyphénols totaux, indique la validité des recommandations sanitaires relatives aux polyphénols dans ledit échantillon d'huile et ladite durée de conservation minimale selon l'étape c) du procédé est la date jusqu'à laquelle l'échantillon d'huile a un niveau de bio-phénols dans 20 g d'huile au moins supérieur ou égal à 5 mg.

9. Appareil (100) adapté pour mettre en œuvre le procédé selon la revendication 1 afin d'évaluer la validité temporelle des recommandations sanitaires relatives aux polyphénols présents dans un échantillon d'huile végétale vierge, **caractérisé en ce qu'**il comprend une première chambre (1) comprenant un papier filtre (11), dans lequel le papier filtre (11) présente soit (A) les composés méta-periodate de sodium (NaIO₄ ) et de 3-méthyl-2-benzothiazolinone hydrazone (MBTH) immobilisés à sa surface, soit (B) lorsque le papier filtre ne comprend pas les composés immobilisés à sa surface, les composés méta-periodate de sodium (NaIO₄) et de 3-méthyl-2-benzothiazolinone hydrazone (MBTH) sont ajoutés séparément dans un tube prédosé comprenant un mélange d'éther éthylique/alcool éthylique et d'eau, l'appareil comprenant en outre :
une chambre de balayage (2) comprenant un dispositif électronique (22) muni d'une caméra (220) choisie parmi un smartphone ou une tablette et dans lequel ledit dispositif électronique (22) comprend en outre un processeur de données (3) configuré pour exécuter l'étape c) du procédé selon la revendication 1.
